(19) Europäisches Patentamt
European Patent Office
Office européen des brevets

(11) Numéro de publication : **0 021 525**
**B1**

(12) **FASCICULE DE BREVET EUROPÉEN**

(45) Date de publication du fascicule du brevet : **29.02.84**

(51) Int. Cl.³ : **C 07 D315/00**, C 07 C 51/285, C 07 C 67/42, C 07 C 53/126

(21) Numéro de dépôt : **80200572.8**

(22) Date de dépôt : **17.06.80**

(54) **Procédé pour la fabrication de composés carboxylés.**

(30) Priorité : **29.06.79 FR 7917307**

(43) Date de publication de la demande : **07.01.81 Bulletin 81/01**

(45) Mention de la délivrance du brevet : **29.02.84 Bulletin 84/09**

(84) Etats contractants désignés : **AT BE CH DE FR GB IT LI LU NL SE**

(56) Documents cités :
**FR-A- 2 372 161**
**GB-A- 753 686**
**GB-A- 1 050 846**
**US-A- 3 457 298**
**Le dossier contient des informations techniques présentées postérieurement au dépôt de la demande et ne figurant pas dans le présent fascicule.**

(73) Titulaire : **INTEROX Société anonyme dite:**
**Rue du Prince Albert, 33**
**B-1050 Bruxelles (BE)**

(72) Inventeur : **Lecloux, André**
**Van Immersseellaan, 15**
**B-1860 Meise (BE)**
Inventeur : **Declerck, Claude**
**Avenue des Croix de Guerre, 128**
**B-1120 Bruxelles (BE)**
Inventeur : **Legrand, Franz**
**Rue des Vaches, 71**
**B-7300 Quaregnon (BE)**

(74) Mandataire : **Meyers, Liliane et al**
**Solvay & Cie Département de la propriété industrielle**
**310, rue de Ransbeek**
**B-1120 Bruxelles (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 021 525 B1**

Jouve, 18, rue St-Denis, 75001 Paris, France

Procédé pour la fabrication de composés carboxylés

La présente invention concerne un procédé pour la fabrication de composés carbonylés tels que des esters, des lactones et des acides carboxyliques à partir des composés carbonylés correspondants. Elle concerne plus particulièrement un procédé pour la fabrication de lactones, et plus spécialement un procédé pour la fabrication de la caprolactone, par réaction des cétones correspondantes avec le peroxyde d'hydrogène.

Les lactones sont en général fabriquées par oxydation des cétones cycliques correspondantes au moyen de peracides carboxyliques. Cette technique, bien que très efficace, nécessite la fabrication préliminaire des peracides, par exemple par oxydation directe de l'aldéhyde correspondant. En outre, la présence dans le milieu réactionnel des peracides carboxyliques et des acides correspondants entraîne la formation de quantités relativement importantes de sous-produits qui en dérivent.

Pour simplifier le schéma réactionnel, on a proposé, dans le brevet britannique 1 050 846 déposé le 16 septembre 1964 au nom de Imperial Chemical Industries Ltd, de faire réagir directement une cétone cyclique, telle que la cyclohexanone, avec le peroxyde d'hydrogène en présence d'un acide organique ou inorganique et d'un catalyseur constitué d'un oxyacide d'un élément d'une période longue des Groupes IV et VI du Tableau périodique des Eléments. Les quantités de catalyseurs mises en œuvre sont cependant extrêmement élevées. En outre, les rendements en caprolactone de ce procédé connu sont très faibles, vraisemblablement du fait de la formation d'acide hydroxycaproïque. Or, ce produit, qui ne peut pas facilement se transformer en caprolactone, se révèle très gênant lors de l'utilisation de la caprolactone pour la fabrication de polymères tels que les polyesters, les polyols, et les polyuréthanes.

On a également proposé, dans le brevet US-A-3 457 298 (UNIVERSAL OIL PRODUCTS Co), de transformer des cétones en esters correspondants en les faisant réagir directement avec le peroxyde d'hydrogène en présence de fluorure d'hydrogène comme catalyseur. Celui-ci est mis en œuvre en quantités tellement élevées qu'en pratique, il devient le solvant de réaction de sorte que pour récupérer l'ester, on doit recourir à de nombreuses opérations de séparation.

De même, dans le brevet GB-A-753 686 (CELANESE CORPORATION OF AMERICA), on propose de transformer une cétone cyclique en lactone correspondante en la faisant réagir avec le peroxyde d'hydrogène en présence d'acide sulfurique comme catalyseur et de traiter la lactone obtenue par de l'acide chlorhydrique pour former l'acide ω-chlorocarboxylique correspondant. Ici aussi, les quantités de catalyseurs mises en œuvre sont extrêmement élevées. En outre, les durées de réaction sont très grandes et il faut prendre des précautions pour éviter la formation de péroxydes indésirables.

La présente invention a pour but de fournir un procédé pour la fabrication de composés carboxylés qui permet de pallier les inconvénients précités des procédés connus et notamment d'obtenir le composé carboxylé avec une bonne sélectivité et en une étape, sans préparation préalable de réactifs intermédiaires tels que les peracides carboxyliques.

Le procédé selon l'invention permet d'éviter une désactivation des catalyseurs. De ce fait, ceux-ci peuvent être mis en œuvre en quantités réduites et ils ont des durées de vie très élevées.

Le procédé selon l'invention permet en outre d'atteindre des vitesses réactionnelles très élevées ce qui permet d'accroître sensiblement la productivité des réacteurs. De plus, le procédé permet d'éliminer aisément la chaleur de réaction, et il nécessite des consommations en énergie réduites. Par ailleurs, la séparation du mélange réactionnel résultant du procédé en ses divers constituants est fortement simplifiée du fait qu'il n'y a pas d'acide carboxylique sous-produit.

Le procédé selon l'invention peut aussi être réalisé aisément en continu et dans de bonnes conditions de sécurité, car il n'exige pas la mise en œuvre de solutions fortement concentrées en composés peroxydés.

L'invention concerne à cet effet un procédé pour la fabrication de composés carboxylés par réaction du composé carbonylé correspondant avec le peroxyde d'hydrogène en milieu liquide et en présence d'un catalyseur, selon lequel le composé carbonylé est choisi parmi les cétones et aldéhydes substitués ou non, saturés ou non, de type aliphatique ou alicyclique contenant de 4 à 30 atomes de carbone et le composé carboxylé est un acide carboxylique lorsque le composé carbonylé est un aldéhyde, un ester lorsque le composé carbonylé est une cétone et une lactone lorsque le composé carbonylé est une cétone cyclique, selon lequel on met en œuvre un catalyseur de Friedel-Crafts qui est un acide de Lewis en quantité comprise entre 0,001 et 50 g par kg de mélange réactionnel et on maintient dans le mélange réactionnel moins de 5 % en poids d'eau, en éliminant en continu l'eau venant à s'y trouver par vaporisation. De préférence, on maintient dans le mélange une concentration en eau inférieure à 2 % de son poids. De très bons résultats ont été obtenus lorsque le mélange réactionnel contient moins de 1 % en poids d'eau.

Pour maintenir le mélange à l'état substantiellement anhydre, on élimine en continu l'eau venant à s'y trouver. Il s'agit en général de l'eau formée par la réaction et de l'eau introduite éventuellement avec les réactifs. Pour ce faire, on peut utiliser diverses techniques. L'eau présente dans le mélange réactionnel est éliminée par des procédés de vaporisation tels que la distillation, la distillation azéotropique ou l'entraînement au moyen d'un gaz inerte.

Si l'eau forme avec un des constituants du mélange, tel que le composé carbonylé ou le solvant

éventuel, un azéotrope à minimum dont la température d'ébullition est plus faible que celle des autres constituants du mélange et des autres azéotropes éventuels qui pourraient se former, on élimine en général l'eau par distillation azéotropique. Cette technique convient tout particulièrement bien lorsque l'azéotrope ainsi formé est un azéotrope hétérogène, car il est alors possible de recycler la phase organique dans le mélange réactionnel, après séparation de la phase aqueuse du distillat.

Lorsque la température d'ébullition de l'eau est plus faible que celle des autres constituants du mélange réactionnel et des azéotropes éventuels qui pourraient se former, on utilise le plus souvent un procédé de distillation ou un procédé d'entraînement de l'eau par passage en continu d'un gaz inerte dans le mélange réactionnel. Cette dernière technique est en général utilisée lorsqu'on veut éviter de porter à l'ébullition des mélanges susceptibles de se décomposer à leur température d'ébullition.

Le procédé selon l'invention est appliqué à des composés carbonylés contenant de 4 à 30 atomes de carbone et de préférence de 5 à 26 atomes de carbone. Les composés carbonylés peuvent contenir une ou plusieurs fonctions carbonyles. Le procédé peut ainsi être appliqué avantageusement à des cyclopentanones et cyclohexanones, substituées ou non, telles que la cyclopentanone, la benzylidènecyclopentanone, la cyclohexanone, la bromocyclohexanone, la chlorocyclohexanone, la méthylcyclohexanone et la benzylidènecyclohexanone, à d'autres cétones cycliques telles que la cyclohexènone, la cyclooctanone, l'isophorone, la fluorenone, la cyclododécanone, la bicyclooctanone, la méthylbenzoquinone, le norcamphre, les naphtoquinones, la muscone et le camphre, à des méthylcétones telles que la méthylcyclobutylcétone, la méthylcycloheptylcétone, la méthyl-n-hexylcétone, la méthylcyclopropylcétone, la méthylisobutylcétone, l'acétophénone, l'acétylméthylcyclopentane, l'acétylméthylcyclohexane et la pinacolone, à d'autres cétones linéaires telles que la nitrobenzophénone, la benzophénone, la méthoxybenzophénone, la cyclohexylphénylcétone, les cétostéroïdes (composés à squelette cyclopentanophénanthrénique substitué) et les cétones-alcools, ainsi qu'à des aldéhydes telles que la vanilline, l'heptanal, le benzaldéhyde et les aldoses.

Le procédé selon l'invention convient bien pour l'oxydation de cétones. Il convient particulièrement bien pour l'oxydation de cétones cycliques, c'est-à-dire de cétones dans lesquelles la fonction carbonyle fait partie du cycle.

De bons résultats ont été obtenus lors de l'application du procédé selon l'invention aux cyclohexènones, aux cyclopentanones, aux méthylcyclohexanones, à l'heptanal, et, tout spécialement, à la cyclohexanone.

Le peroxyde d'hydrogène peut être mis en œuvre dans le procédé selon l'invention sous forme de solutions aqueuses ou de solutions dans un solvant organique. Pour des raisons économiques, on utilise en général des solutions aqueuses de peroxyde d'hydrogène. Celles-ci peuvent avoir des concentrations très variables en peroxyde d'hydrogène. En général les solutions aqueuses de peroxyde d'hydrogène contiennent plus de 10 % en poids du peroxyde d'hydrogène. Des concentrations inférieures sont moins souvent utilisées car elles nécessitent l'élimination de quantités importantes d'eau. Les solutions ne contiennent en général pas plus de 90 % de peroxyde d'hydrogène. Des solutions ayant une concentration supérieure peuvent être utilisées mais elles sont difficiles à produire et assez dangereuses à utiliser. On utilise de préférence des solutions contenant de 20 à 85 % en poids de peroxyde d'hydrogène.

Dans le mélange réactionnel mis en œuvre selon l'invention, les proportions de réactifs peuvent varier dans de très larges limites en fonction des vitesses d'introduction choisies pour les réactifs, de la vitesse de réaction, de la mise en œuvre éventuelle d'un solvant inerte, et de l'élimination éventuelle de l'eau par distillation d'un azéotrope eau-composé carbonylé. Ainsi le nombre de moles de peroxyde d'hydrogène dans le mélange réactionnel ne dépasse en général pas deux fois le nombre de moles de fonctions carbonyles. De bons résultats ont été obtenus lorsqu'on maintient dans le mélange réactionnel un défaut de peroxyde d'hydrogène par rapport au composé carbonylé de manière à réaliser un rapport molaire peroxyde d'hydrogène sur composé carbonylé ne dépassant pas 0,9.

Dans le mélange réactionnel, on maintient en général une proportion de peroxyde d'hydrogène faible qui peut descendre jusqu'à 0,000 1 % en poids et même en dessous. En début de réaction, la proportion de peroxyde d'hydrogène présente dans le mélange réactionnel mis en œuvre est en général comprise entre 0,001 et 10 % de son poids et la proportion de composé carbonylé est en général comprise entre 5 et 99,9 % en poids. Si le mélange réactionnel mis en œuvre ne comporte pas de solvant inerte cette dernière proportion est le plus souvent comprise entre 90 et 99,9 % en poids.

Quant au rapport molaire de peroxyde d'hydrogène par rapport au composé carbonylé frais (à l'exclusion du produit de recyclage) envoyé au réacteur, il est en général voisin de la stœchiométrie et il est le plus souvent compris entre 0,5 et 1,5.

Les catalyseurs utilisés dans le procédé selon l'invention sont choisis parmi les catalyseurs de Friedel-Crafts, et leurs mélanges, qui sont des acides de Lewis. En général, on utilise comme catalyseurs des composés de métaux choisis parmi le beryllium, le magnésium, les éléments des Groupes IIb, IIIa et b, IVb, Vb, VIb et VIII et les éléments des Périodes 3, 4, 5 et 6 des Groupes IVa, Va et VIa, ou du fluorure d'hydrogène. De bons résultats ont été obtenus avec des composés de métaux choisis parmi le beryllium, le zinc, le cadmium, le bore, l'aluminium, le gallium, l'indium, le sandium, l'yttrium, le lanthane, le silicium, le germanium, l'étain, le titane, le zirconium, l'hafnium, le thorium, l'antimoine, le bismuth, le vanadium, le niobium, le tantale, le tellure, le chrome, le molybdène, le tungstène, le fer et le ruthénium. Les composés du titane, du tantale, du tungstène, de l'étain, du molybdène, de l'antimoine, du bore et du

*zinc ont donné des résultats excellents.*

Les composés de métaux utilisés comme catalyseurs peuvent être de natures diverses. Ils sont en général choisis parmi les fluorures, les chlorures, les oxychlorures, les oxyfluorures, les perchlorates et les fluoroalkanesulfonates ainsi que leurs complexes.

Les catalyseurs préférés sont ceux qui sont susceptibles de former des complexes du type « acide-base de Lewis » avec le composé carbonylé à oxyder et qui ne sont pas trop vite hydrolysés dans les conditions de réaction, de manière à avoir une durée de vie nettement supérieure au temps de séjour moyen des réactifs dans le réacteur.

Les meilleurs résultats ont été obtenus avec les fluorures de titane, de tantale, de tungstène, d'étain, d'antimoine et de zinc, les chlorures d'étain, le perchlorate de zinc et le fluorure de bore complexé par un éther tel que $BF_3 \cdot O(C_2H_5)_2$, dont l'utilisation est dès lors préférée.

Les catalyseurs mis en œuvre peuvent être éventuellement préparés in situ par réaction de l'oxyde métallique du métal, des alkyl-métaux, des alkoxydes métalliques ou en général des sels métalliques correspondants. Ainsi, lors de l'emploi de fluorures métalliques, on peut introduire dans le mélange réactionnel simultanément du fluorure d'hydrogène et un oxyde du métal choisi. Cette technique se révèle avantageuse lorsque le fluorure métallique choisi comme catalyseur est difficile à dissoudre dans le mélange réactionnel. Tel est le cas pour les fluorures de beryllium et de zinc par exemple.

Les catalyseurs mis en œuvre peuvent être éventuellement fixés sur un support organique ou inorganique.

La quantité de catalyseur mise en œuvre est choisie de manière à assurer une concentration stationnaire suffisante pour catalyser la réaction. De bons résultats sont obtenus lorsque le catalyseur est mis en œuvre à raison de 0,01 à 20 g par kg de mélange réactionnel.

Lorsque le catalyseur est constitué de fluorure d'hydrogène les quantités de catalyseur nécessaires sont en général plus importantes.

Les catalyseurs peuvent être introduits de diverses manières connues en elles-mêmes. On peut ainsi procéder à une introduction unique, une introduction continue ou une introduction étagée de catalyseurs. Le catalyseur peut être mis en œuvre à l'état pur. Cependant il est avantageux de le mettre en œuvre sous forme d'une solution dans un des constituants du mélange réactionnel.

Outre les réactifs et les catalyseurs, le mélange réactionnel peut également contenir, sans que cela soit indispensable, un ou plusieurs solvants inertes dans les conditions de réaction. Divers types de solvants inertes peuvent être utilisés. Ils peuvent être choisis parmi les éthers, les alcools, les hydrocarbures halogénés, les hydrocarbures non substitués, les esters carboxyliques, les esters non acides d'acide phosphorique, les hydrocarbures substitués par des groupes nitro, ainsi que certaines cétones moins réactives vis-à-vis du peroxyde d'hydrogène dans les conditions de réaction, telle que l'acétone.

Comme hydrocarbures halogénés convenant bien en général on peut signaler les hydrocarbures halogénés aromatiques, aliphatiques et alicycliques contenant de 1 à 10 atomes de carbone dans leur molécule substitués par au moins un halogène choisi de préférence parmi le chlore et le fluor et pouvant être en outre substitués par un groupe hydroxyle.

Comme hydrocarbures non substitués convenant bien en général on peut signaler les hydrocarbures contenant de 5 à 14 atomes de carbone aliphatiques, aromatiques ou alicycliques.

Comme éthers convenant bien en général on peut signaler les éthers aliphatiques ou alicycliques, symétriques ou asymétriques, contenant de 3 à 20 et de préférence de 4 à 12 atomes de carbone tels que le diéthyléther, le diphényléther, les diméthoxy -mono- ou -di-éthylèneglycols, le tétrahydrofurane et le diisopropyléther.

Comme alcools convenant bien en général on peut signaler les alcools mono- ou poly-hydriques, primaires, secondaires ou tertiaires contenant de 1 à 10 atomes de carbone tels que l'éthanol et le cyclohexanol.

Comme esters carboxyliques convenant bien en général, on peut mentionner les esters aliphatiques contenant de 3 à 9 atomes de carbone dans la molécule.

Comme hydrocarbures substitués par des groupes nitro convenant bien en général, on peut mentionner les hydrocarbures contenant de 1 à 10 atomes de carbone tels que le nitrométhane et le nitrobenzène.

Quant aux esters d'acide phosphorique pouvant convenir, ils répondent en général à la formule

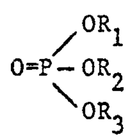

où $R_1$, $R_2$, $R_3$ sont identiques ou différents et représentent des groupes alkyles ou aryles, substitués ou non, tels que la molécule contient de 3 à 30 atomes de carbone. A titre d'exemples de phosphates particuliers, on peut signaler les phosphates de triéthyle et de trioctyle.

Lorsque l'eau est difficile à séparer du mélange réactionnel on peut incorporer au mélange un

solvant capable de former un azéotrope à minimum avec l'eau. Dans ce cas, on utilise en général un solvant capable de former un azéotrope hétérogène avec l'eau, de manière à pouvoir séparer du distillat de la distillation azéotropique, la phase organique que l'on peut recycler au réacteur.

Les solvants éventuels sont présents dans des proportions variables pouvant varier de 0 à 95 % du poids du mélange réactionnel. Quand on met en œuvre des solvants, en général ils sont présents à raison de 30 à 95 % du poids du mélange réactionnel. Lors de l'oxydation de la cyclohexanone on n'utilise en général pas de solvants inertes.

On peut également ajouter au mélange réactionnel d'autres additifs tels que des agents de stabilisation du peroxyde d'hydrogène, des inhibiteurs de polymérisation ou des dérivés minéraux ou organiques susceptibles de fixer l'eau du milieu réactionnel. Ces additifs éventuels sont en général présents à raison de moins de 10 % du poids du mélange réactionnel.

La température et la pression de réaction peuvent varier dans de très larges limites. Elles sont choisies en fonction de la nature du composé carbonylé à oxyder. En général on les règle de manière à assurer l'ébullition et à ne pas dépasser la température de décomposition du mélange réactionnel. La température est habituellement inférieure à 423 K et le plus souvent comprise entre 293 et 393 K. De bons résultats ont été obtenus à des températures comprises entre 313 et 373 K. La pression est en général inférieure à $5.10^5$ Pa. De bons résultats ont été obtenus en utilisant des pressions comprises entre $1.10^3$ Pa et $3.10^5$ Pa.

Le procédé selon l'invention peut être mis en œuvre en continu ou en discontinu dans un réacteur unique ou dans une série de réacteurs en parallèle ou consécutifs. Pour réaliser le procédé selon l'invention on peut utiliser n'importe quel appareillage convenant pour les mélanges réactionnels liquides. Plus particulièrement, il est avantageux d'utiliser des réacteurs, connus en eux-mêmes, permettant la distillation en cours de réaction de constituants d'un mélange réactionnel liquide. Ces réacteurs sont avantageusement couplés à des colonnes de distillation connues en elles-mêmes.

Le procédé selon l'invention peut être réalisé en continu dans un appareil tel que celui représenté schématiquement à la figure unique du dessin en annexe qui se rapporte à un mode de réalisation pratique particulier.

Cet appareil convient tout particulièrement bien pour l'oxydation d'un composé carbonylé susceptible de former un azéotrope hétérogène avec l'eau comme c'est le cas pour la cyclohexanone.

Dans un réacteur 1 surmonté d'une colonne de distillation 2, on introduit par la voie 3 une solution concentrée de peroxyde d'hydrogène et par la voie 4 le composé carbonylé contenant le catalyseur. Le composé carbonylé frais est introduit par la voie 5.

En cours de réaction l'azéotrope eau-composé carbonylé quitte la colonne de distillation 2 par la voie 9, est condensé dans le condenseur 10, et est envoyé par la voie 11 au décanteur 12. Lorsque le composé carbonylé a une densité inférieure à celle de l'eau, on le recueille en tête du décanteur par la voie 13 tandis que l'eau est recueillie en pied du décanteur par la voie 14 ; dans le cas contraire, les prélèvements sont inversés. Le composé carbonylé est recyclé à la colonne de distillation par la voie 15 où il constitue le reflux. Dans certains cas, on peut envoyer une partie du composé carbonylé par la voie 16 dans le mélangeur 6 qui est alimenté par la voie 5 en composé carbonylé frais et en catalyseur.

On soutire en continu par la voie 7 une partie du mélange réactionnel que l'on soumet à des séparations successives pour obtenir d'une part le composé carbonylé non transformé que l'on renvoie en 6 et d'autre part le composé carboxylé qui constitue la production.

Le mélange soutiré par la voie 7 peut éventuellement être soumis à un traitement préliminaire de manière à permettre la séparation du catalyseur qui pourrait s'y trouver. On peut ainsi ajouter au mélange une base de Lewis forte de manière à complexer le catalyseur. Des bases de Lewis convenant pour cet usage sont par exemple la pyridine et la 8-hydroxyquinoléine. Le complexe ainsi formé est séparé du mélange et peut ensuite être soumis à diverses étapes connues en elles-mêmes de régénération du catalyseur telles qu'une décomposition thermique. Le catalyseur régénéré peut être recyclé au réacteur de fabrication du composé carboxylé.

L'appareil représenté à la figure 1 peut également être utilisé pour réaliser le procédé de l'invention en présence d'un solvant capable de former avec l'eau un azéotrope hétérogène dont la température d'ébullition est inférieure à celle de tous les constituants et azéotropes éventuels du mélange réactionnel. Dans ce cas la phase organique recueillie dans le décanteur 12 est constituée du solvant de réaction.

Les composés carboxylés obtenus selon le procédé de l'invention peuvent être utilisés pour la fabrication de polymères tels que des polyesters, des polyols ou des polyuréthanes.

Afin d'illustrer l'invention, sans pour autant en limiter la portée, on donne ci-après des exemples de fabrication de composés carboxylés (exemples 1 à 33, 35, 37 à 49). Les exemples 34 et 36 sont donnés à titre de comparaison.

## Exemple 1

La cyclohexanone est introduite dans un réacteur en verre à double enveloppe chauffé par circulation d'huile et surmonté d'un réfrigérant maintenu à 263 K. On ajoute du $SbF_5$ à raison de 0,2 g/kg de cyclohexanone (CHO) puis l'on porte et l'on maintient la température à 363 K sous une pression de $2.10^4$ Pa afin de distiller l'azéotrope aqueux. Par l'intermédiaire d'un tube capillaire on admet alors de

l'$H_2O_2$ à 95 % au débit de 130 ml/h.l. Les produits de distillation sont recueillis et dosés par chromatographie, comme les produits de réaction. La productivité en ε-caprolactone (ε-CL) est de 221 g/h.l avec une sélectivité par rapport à $H_2O_2$ de 73 %.

Exemples 2 à 16

Ces essais sont réalisés de façon analogue à l'exemple 1.

La température et la pression sont respectivement de 353 K et 1,33.10$^4$ Pa, et l'$H_2O_2$ à 95 % est dilué par un mélange acétone-cyclohexanone dans les proportions volumétriques 5/50/30. Le débit de $H_2O_2$ 95 % est de 67 ml/h.l. La durée de l'essai est de 30 minutes environ. Les autres conditions opératoires et les résultats obtenus sont repris au Tableau I.

Tableau I

| Essai n° | Catalyseur | | Productivité, g ε-CL/h.l | Sélectivité par rapport à $H_2O_2$, % |
|---|---|---|---|---|
| | nature | concentration, g/kg CHO | | |
| 2 | $SbF_5$ | 0,04 | 242 | 80 |
| 3 | $SbF_5$* | 0,4 | 208 | 69 |
| 4 | $SbCl_5$ | 0,5 | 81 | 41 |
| 5 | $SnCl_4$ | 0,2 | 190 | 63 |
| 6 | $BF_3.O(C_2H_5)_2$ | 1,2 | 242 | 80 |
| 7 | $SnF_4$ | 1,4 | 133 | 45 |
| 8 | $MoOCl_3$.9,10-P.Q.** | 0,4 | 189 | 62 |
| 9 | $SbF_3$ | 1,4 | 231 | 76 |
| 10 | $BF_3$ | 1,1 | 222 | 73 |
| 11 | $TiF_4$ | 0,7 | 169 | 56 |
| 12 | $MoO_2Cl_2$ | 0,4 | 187 | 62 |
| 13 | $MoCl_5$ | 0,2 | 185 | 61 |
| 14 | $BF_3.CH_3OH$ | 0,4 | 149 | 49 |
| 15 | $TaF_5$ | 0,4 | 164 | 54 |
| 16 | HF | 2,7 | 211 | 70 |

* le pentafluorure d'antimoine est déposé sur graphite (vendu par la firme ALDRICH sous la marque GRAPHIMET) à raison de 47 % du poids total de catalyseur.

** P.Q. = Phénanthrènequinone.

Avec le $SbF_5$, la sélectivité moyenne par rapport à la cyclohexanone est de 82 %.

Exemples 17 à 28

Ces essais sont réalisés de façon analogue à l'exemple 1.

La température et la pression sont respectivement de 353 K et 1,6.10$^4$ Pa et l'$H_2O_2$ 84 % est dilué par un mélange tétrahydrofuranecyclohexanone dans les proportions volumétriques 6/50/30. Le débit de $H_2O_2$ 84 % est de 80 ml/h.l. Les autres conditions opératoires et les résultats obtenus sont repris au Tableau II.

(Voir Tableau II, page 7)

Tableau II

| Essai n° | Catalyseur | | Productivité, g ε-CL/h.l | Sélectivité par rapport à $H_2O_2$, % | Sélectivité par rapport à la CHO, % |
|---|---|---|---|---|---|
| | Nature | Concentration g/kg CHO | | | |
| 17 | $SbF_5$ | 0,079 | 231 | 75,9 | 84,7 |
| 18 | $SnCl_4$ | 0,3 | 209 | 68,8 | 91,7 |
| 19 | $BF_3.O(C_2H_5)_2$ | 1,2 | 242 | 79,5 | 78,3 |
| 20 | $SnF_4$ | 1,4 | 132 | 43,4 | 69,9 |
| 21 | $SbF_3$ | 1,4 | 234 | 76,9 | 92,1 |
| 22 | $BF_3$ | 0,7 | 233 | 76,7 | 90,8 |
| 23 | $TiF_4$ | 0,7 | 163 | 53,5 | 96,4 |
| 24 | $MoO_2Cl_2$ | 0,2 | 168 | 55,2 | 87,5 |
| 25 | $MoCl_5$ | 0,2 | 174 | 57,3 | 91,6 |
| 26 | $TaF_5$ | 0,4 | 222 | 73 | 91,3 |
| 27 | $WF_6$ | 0,035ml* | 236 | 77,5 | 84,7 |
| 28 | HF | 2,7 | 231 | 75,9 | 89,8 |

* La concentration est donnée en ml/kg CHO.

Exemples 29 à 32

Ces essais sont réalisés de façon analogue à l'exemple 1.

Les catalyseurs ont été formés in situ par mélange de l'oxyde métallique avec HF dans la cyclohexanone.

La température et la pression sont respectivement de 353 K et $1,5.10^4$ Pa et l'$H_2O_2$ 95 % est dilué par un mélange acétone-cyclohexanone dans les proportions volumétriques 5/50/30. Le débit de $H_2O_2$ 95 % est de 67 ml/h.l. Les autres conditions opératoires et les résultats obtenus sont repris au Tableau III.

Tableau III

| Essai n° | Catalyseur | | Productivité, g ε-CL/h.l | Sélectivité par rapport à $H_2O_2$, % |
|---|---|---|---|---|
| | nature | Poids, g/kg CHO | | |
| 29 | HF/ZnO | 0,3/0,3 | 189 | 62 |
| 30 | $HF/Sb_2O_3$ | 0,3/0,7 | 224 | 74 |
| 31 | $HF/Sb_2O_5$ | 0,3/0,5 | 222 | 73 |
| 32 | HF/ZnO | 2,7/3,4 | 235 | 77 |

Exemples 33, 34, 35 et 36

L'influence de l'élimination azéotropique de l'eau sur les performances des catalyseurs est illustrée ci-dessous.

Les essais 34 et 36 sans élimination d'eau sont réalisés suivant l'exemple 1 mais avec un reflux total. La température et la pression sont respectivement de 353 K et $1.10^5$ Pa.

Les essais 33 et 35 avec distillation de l'azéotrope sont réalisés suivant le mode opératoire des exemples 2 à 16.

Dans tous les cas, le débit de $H_2O_2$ 95 % est de 67 ml/h.l. Les autres conditions opératoires et les résultats obtenus sont repris au Tableau IV.

Tableau IV

| Essai n° | Catalyseur | | Elimination de $H_2O$ | Productivité, g ε-CL/h.l | Sélectivité par rapport à $H_2O_2$, % |
|---|---|---|---|---|---|
| | Nature | Concentration g/kg CHO | | | |
| 33 | HF | 2,7 | oui | 211 | 70 |
| 34 | HF | 2,7 | non | 65* | 28* |
| 35 | $SbF_5$ | 0,04 | oui | 242 | 80 |
| 36 | $SbF_5$ | 0,04 | non | 78* | 33* |

* Pour ces deux essais, le taux de décomposition de $H_2O_2$ est inférieur à 100 %. Ces valeurs sont donc entachées d'erreur (erreur par excès).

Exemple 37

Cet essai est réalisé dans les conditions des exemples 17 à 28 en utilisant $SbF_5$ comme catalyseur, 0,2 g/kg de cyclohexanone et de l'$H_2O_2$ à 70 %. La température et la pression sont respectivement de 355 K et $1,6.10^4$ Pa et l'$H_2O_2$ à 70 % est dilué par un mélange tétrahydrofurane-cyclohexanone dans les proportions volumétriques 7,5/50/30.

Le débit de $H_2O_2$ 70 % est de 100 ml/h.l.

La productivité en ε-caprolactone est de 225 g/h.l avec une sélectivité par rapport à $H_2O_2$ de 74 %.

Exemple 38

Cet essai est réalisé dans des conditions analogues à celles des exemples 17 à 28. La concentration du catalyseur, $SbF_5$, est de 0,3 g/kg de cyclohexanone. La température et la pression sont respectivement de 331 K et $5,3.10^3$ Pa et l'$H_2O_2$ à 87 % est dilué par un mélange tétrahydrofurane-cyclohexanone dans les proportions volumétriques 6/50/30.

Le débit de $H_2O_2$ 87 % est de 80 ml/h.l.

La productivité en ε-caprolactone est de 214 g/h.l avec une sélectivité par rapport à $H_2O_2$ de 67 %.

Exemple 39

Cet essai est réalisé dans des conditions analogues à celles des essais 17 à 28. La cétone utilisée est la 2-méthylcyclohexanone, et le catalyseur, $BF_3.O(C_2H_5)_2$, est mis en œuvre à raison de 3,3 g/kg de cétone. La température et la pression sont respectivement de 353 K et $1,5.10^4$ Pa et l'$H_2O_2$ à 84 % est dilué par un mélange tétrahydrofurane-2-méthylcyclohexanone dans les proportions volumétriques 6/50/30.

Le débit de $H_2O_2$ 84 % est de 60 ml/h.l.

La productivité en lactones est de 180 g/h.l avec une sélectivité par rapport à $H_2O_2$ de 71 %.

Exemple 40

Cet essai est réalisé dans des conditions analogues à celles des essais 17 à 28. La cétone utilisée est la cyclopentanone et le catalyseur, $BF_3.O(C_2H_5)_2$, est mis en œuvre à raison de 4,0 g/kg de cétone. La température et la pression sont respectivement de 346 K et $2,9.10^4$ Pa et l'$H_2O_2$ 87 % est dilué par un mélange tétrahydrofurane-cyclopentanone dans les proportions volumétriques 6/50/30.

Le débit de $H_2O_2$ 87 % est de 60 ml/h.l.

La productivité en δ-valérolactone est de 103 g/h.l avec une sélectivité par rapport à $H_2O_2$ de 49 %.

Exemple 41

Cet essai est réalisé dans des conditions analogues à celles des exemples 17 à 28 en utilisant une solution de cyclohexanone dans le diphényléther à 469 g/kg. On utilise le $BF_3.O(C_2H_5)_2$ comme catalyseur (3,2 g/kg de cyclohexanone) et de l'$H_2O_2$ à 87 %. La température et la pression sont respectivement de 383 K et $9,3.10^3$ Pa et l'$H_2O_2$ à 87 % est diluée par un mélange tétrahydrofurane-cyclohexanone dans les proportions volumétriques 6/50/30.

Le débit de $H_2O_2$ à 87 % est de 80 ml/h.l.

La productivité en ε-caprolactone est de 183 g/h.l avec une sélectivité par rapport à $H_2O_2$ de 58 %.

Exemple 42

Cet essai est réalisé dans des conditions de l'essai 41 mais l'on utilise une solution de cyclohexanone dans le 1,1,2,2-tétrachloroéthane à 374 g/kg. La température et la pression sont respectivement de 385 K et $1,3.10^4$ Pa.

La productivité en ε-caprolactone est de 220 g/h.l avec une sélectivité par rapport à $H_2O_2$ de 69 %.

Exemple 43

Cet essai est réalisé dans des conditions analogues à celles de l'exemple 1 mais le volume dans le réacteur est maintenu constant par addition continue de cyclohexanone. On utilise le $SbF_5$ comme catalyseur (0,2 g/kg de cyclohexanone) et de l'$H_2O_2$ à 41 %. La température et la pression sont respectivement de 357 K et $1,4.10^4$ Pa.

Le débit de $H_2O_2$ à 41 % est de 462 ml/h.l.

La productivité en ε-caprolactone est de 489 g/h.l avec une sélectivité par rapport à $H_2O_2$ de 67 %.

Exemple 44

Cet essai est réalisé dans les conditions opératoires de l'exemple 43 mais la cyclohexanone d'addition contient 0,08 g de catalyseur/kg de cyclohexanone et l'$H_2O_2$ utilisé est à 20 %. La température et la pression sont respectivement de 355 K et $1,4.10^4$ Pa.

Le débit de $H_2O_2$ à 20 % est de 244 ml/h.l.

La productivité en ε-caprolactone est de 104 g/h.l avec une sélectivité par rapport à $H_2O_2$ de 57 %.

Exemple 45

Cet essai est réalisé dans des conditions analogues à l'essai 44 mais avec l'$H_2O_2$ à 69 %. La température et la pression sont respectivement de 363 K et $1,4.10^4$ Pa.

Le débit de $H_2O_2$ à 69 % est de 242 ml/h.l.

La productivité en ε-caprolactone est de 534 g/h.l avec une sélectivité par rapport à $H_2O_2$ de 75 %.

Exemple 46

Cet essai est réalisé dans des conditions analogues à l'essai 44 mais avec de l'$H_2O_2$ à 86 %. La température et la pression sont respectivement de 364 K et $1,4.10^4$ Pa.

Le débit de $H_2O_2$ à 86 % est de 171 ml/h.l.

La productivité en ε-caprolactone est de 510/h.l avec une sélectivité par rapport à $H_2O_2$ de 76 % et une sélectivité par rapport à la cyclohexanone de 85 %.

Exemple 47

Cet essai est réalisé dans des conditions analogues à l'essai 43 mais avec de l'$H_2O_2$ à 86 % et en utilisant le $SbF_3$ comme catalyseur (1,4 g/kg de cyclohexanone). La température et la pression sont respectivement de 362 K et $1,3.10^4$ Pa.

Le débit de $H_2O_2$ à 86 % est de 141 ml/h.l.

La productivité en ε-caprolactone est de 420 g/h.l avec une sélectivité par rapport à $H_2O_2$ de 76 % et une sélectivité par rapport à la cyclohexanone de 88 %.

Exemple 48

La 2-cyclohexèn-1-one est introduite dans un réacteur analogue à celui de l'exemple 1. On ajoute du $SbF_5$ à raison de 0,2 g/kg de cétone puis l'on porte et l'on maintient la température à 369 K sous une pression de $8.10^3$ Pa. On admet alors de l'$H_2O_2$ à 86 % au débit de 267 ml/h.l.

La productivité en lactones est de 343 g/h.l avec une sélectivité par rapport à $H_2O_2$ de 34 %.

Exemple 49

L'heptanal est introduit dans un réacteur analogue à celui de l'exemple 1. On ajoute du $BF_3.O(C_2H_5)_2$ à raison de 2,7 g/kg d'aldéhyde puis l'on porte et on maintient la température à 351 K sous une pression de $1.10^4$ Pa. On admet alors de l'$H_2O_2$ à 86 % au débit de 267 ml/h.l.

La productivité en acide heptanoïque est de 671 g/h.l avec une sélectivité par rapport à $H_2O_2$ de 58 %.

Exemple 50

Une solution à 50 % de cyclododécanone dans le 1,1,1,2-tétrachloroéthane est introduite dans un

réacteur analogue à celui de l'exemple 1. On ajoute du $BF_3.O(C_2H_5)_2$ à raison de 4,6 g/kg de cétone puis l'on porte et on maintient la température à 347 K sous une pression de $1{,}0.10^4$ Pa. On admet alors de l'$H_2O_2$ à 86 % au débit de 185 ml/h.l.

Un examen par résonance magnétique nucléaire donne une teneur en lactone de 7 % par rapport à la cyclododécanone.

Exemple 51

La cyclohexanone est introduite dans un réacteur analogue à celui de l'exemple 1. On ajoute du $Zn(ClO_4)_2.6H_2O$ à raison de 7,0 g/kg de cétone puis l'on porte et on maintient la température à 353 K sous une pression de $1{,}1.10^4$ Pa. On admet de l'$H_2O_2$ à 86 % au débit de 218 ml/h.l.

La productivité en ε-caprolactone est de 472 g/h.l avec une sélectivité par rapport à $H_2O_2$ de 55 %.

**Revendications**

1. Procédé pour la fabrication de composés carboxylés par réaction du composé carbonylé correspondant avec le peroxyde d'hydrogène en milieu liquide et en présence d'un catalyseur, selon lequel le composé carbonylé est choisi parmi les cétones et aldéhydes substitués ou non, saturés ou non, de type aliphatique ou alicyclique contenant de 4 à 30 atomes de carbone et le composé carboxylé est un acide carboxylique lorsque le composé carbonylé est un aldéhyde, un ester lorsque le composé carbonylé est une cétone et une lactone lorsque le composé carbonylé est une cétone cyclique, caractérisé en ce qu'on met en œuvre un catalyseur de Friedel-Crafts qui est un acide de Lewis en quantité comprise entre 0,001 et 50 g par kg de mélange réactionnel et on maintient dans le mélange réactionnel moins de 5 % en poids d'eau, en éliminant en continu l'eau venant à s'y trouver par vaporisation.

2. Procédé selon la revendication 1, caractérisé en ce qu'on maintient dans le mélange réactionnel moins de 2 % en poids d'eau.

3. Procédé selon la revendication 1 ou 2, caractérisé en ce que l'on choisit le catalyseur parmi le fluorure d'hydrogène et les composés des métaux choisis parmi le beryllium, le zinc, le cadmium, le bore, l'aluminium, le gallium, l'indium, le scandium, l'yttrium, le lanthane, le silicium, le germanium, l'étain, le titane, le zirconium, l'hafnium, le thorium, l'antimoine, le bismuth, le vanadium, le niobium, le tantale, le tellure, le chrome, le molybdène, le tungstène, le fer et le ruthénium, ainsi que leurs mélanges.

4. Procédé selon la revendication 3, caractérisé en ce que l'on choisit le catalyseur parmi les fluorures d'antimoine, de titane, de tantale, de tungstène, d'étain et de zinc, le perchlorate de zinc, le chlorure d'étain et le fluorure de bore complexé par un éther.

5. Procédé selon l'une quelconque des revendications 1 à 4, caractérisé en ce que l'on met en œuvre un mélange réactionnel contenant de 0,001 à 10 % en poids de peroxyde d'hydrogène et de 5 à 99,9 % en poids de composé carbonylé.

6. Procédé selon l'une quelconque des revendications 1 à 5, caractérisé en ce que le mélange réactionnel contient en outre un solvant.

7. Procédé selon l'une quelconque des revendications 1 à 6, caractérisé en ce qu'il est appliqué à la fabrication d'ε-caprolactone par réaction de cyclohexanone.

8. Procédé selon la revendication 7, caractérisé en ce que l'on élimine l'eau du mélange réactionnel par distillation de l'azéotrope eau-cyclohexanone, en ce que l'on condense le distillat et on le soumet à une décantation pour en séparer la phase aqueuse de la phase organique, et en ce que l'on renvoie la phase organique dans le mélange réactionnel.

**Claims**

1. Process for the preparation of carboxyl compounds by reaction of the corresponding carbonyl compound with hydrogen peroxide in a liquid medium and in the presence of a catalyst, according to which the carbonyl compound is selected from amongst substituted or unsubstituted, saturated or unsaturated ketones and aldehydes, of aliphatic or alicyclic type, containing from 4 to 30 carbon atoms, and the carboxyl compound is a carboxylic acid when the carbonyl compound is an aldehyde, an ester when the carbonyl compound is a ketone and a lactone when the carbonyl compound is a cyclic ketone, characterised in that a Friedel-Crafts catalyst, which is a Lewis acid, is used in an amount of between 0.001 and 50 g per kg of reaction mixture, and a water concentration of less than 5 % by weight is maintained in the reaction mixture, the water which has been produced by vaporisation being removed continuously.

2. Process according to Claim 1, characterised in that a water concentration of less than 2 % by weight is maintained in the reaction mixture.

3. Process according to Claims 1 or 2, characterised in that the catalyst is selected from amongst hydrogen fluoride and the compounds of metals selected from amongst beryllium, zinc, cadmium, boron,

aluminium, gallium, indium, scandium, yttrium, lanthanum, silicon, germanium, thin, titanium, zirconium, hafnium, thorium, antimony, bismuth, vanadium, niobium, tantalum, tellurium, chromium, molybdenum, tungsten, iron and ruthenium, as well as mixtures of the above.

4. Process according to Claim 3, characterised in that the catalyst is selected from amongst the fluorides of antimony, titanium, tantalum, tungsten, tin and zinc, zinc perchlorate, tin chloride and complexes of boron fluoride with an ether.

5. Process according to any one of Claims 1 to 4, characterised in that a reaction mixture containing from 0.001 to 10 % by weight of hydrogen peroxide and from 5 to 99.9 % by weight of carbonyl compound is employed.

6. Process according to any one of Claims 1 to 5, characterised in that the reaction mixture furthermore contains a solvent.

7. Process according to any one of Claims 1 to 6, characterised in that it is applied to the preparation of ε-caprolactone by reaction of cyclohexanone.

8. Process according to Claim 7, characterised in that the water is removed from the reaction mixture by distillation of a water-cyclohexanone azeotrope, the distillate is condensed and allowed to settle out so that the aqueous phase can be separated from the organic phase, and the organic phase is recycled to the reaction mixture.

**Ansprüche**

1. Verfahren zur Herstellung carboxylierter Verbindungen durch Umsetzung der entsprechenden Carbonylverbindung mit Wasserstoffperoxyd in flüssigem Milieu und in Anwesenheit eines Katalysators, wobei die Carbonylverbindung ausgewählt ist unter den substituierten oder nicht substituierten, gesättigten oder nicht gesättigten, aliphatischen oder alicyclischen Ketonen und Aldehyden mit 4 bis 30 Kohlenstoffatomen und wobei die carboxylierte Verbindung eine Carbonsäure ist, wenn die Carbonylverbindung ein Aldehyd ist, bzw. ein Ester, wenn die Carbonylverbindung ein Keton ist, bzw. ein Lacton, wenn die Carbonylverbindung ein cyclisches Keton ist, dadurch gekennzeichnet, daß man einen Friedel-Crafts Katalysator, der eine Lewis-Säure ist, einsetzt in einer Menge von 0,001 bis 50 g je kg Reaktionsmischung und man in der Reaktionsmischung weniger als 5 Gew.-% Wasser aufrechterhält, indem man kontinuierlich das Wasser durch Verdampfung entfernt.

2. Verfahren gemäß Anspruch 1, dadurch gekennzeichnet, daß man in der Reaktionsmischung wenigers 2 Gew.-% Wasser aufrechterhält.

3. Verfahren gemäß Anspruch 1 oder 2, dadurch gekennzeichnet, daß man den Katalysator auswählt unter Fluorwasserstoff und den Verbindungen der Metalle Beryllium, Zink, Cadmium, Bor, Aluminium, Gallium, Indium, Scandium, Yttrium, Lanthan, Silicium, Germanium, Zinn, Titan, Zirkonium, Hafnium, Thorium, Antimon, Wismut, Vanadium, Niob, Tantal, Tellur, Chrom, Molybdän, Wolfram, Eisen und Ruthenium sowie deren Mischungen.

4. Verfahren gemäß Anspruch 3, dadurch gekennzeichnet, daß man den Katalysator auswählt unter den Fluoriden von Antimon, Titan, Tantal, Wolfram, Zinn und Zink, Zinkperchlorat, Zinnchlorid und einem mit einem Äther komplexierten Borfluorid.

5. Verfahren gemäß einem der Ansprüche 1 bis 4, dadurch gekennzeichnet, daß man eine Reaktionsmischung einsetzt, die 0,001 bis 10 Gew.-% Wasserstoffperoxyd und 5 bis 99,9 Gew.-% der Carbonylverbindung enthält.

6. Verfahren gemäß einem der Ansprüche 1 bis 5, dadurch gekennzeichnet, daß die Reaktionsmischung außerdem ein Lösungsmittel enthält.

7. Verfahren gemäß einem der Ansprüche 1 bis 6, dadurch gekennzeichnet, daß es angewandt wird auf die Herstellung von. ε-Caprolacton durch Umsetzung von Cyclohexanon.

8. Verfahren gemäß Anspruch 7, dadurch gekennzeichnet, daß man das Wasser aus der Reaktionsmischung entfernt durch Destillation des Azeotrops Wasser-Cyclohexanon, daß man das Destillat kondensiert und einer Dekantierung unterwirft um die wässrige Phase abzutrennen von der organischen Phase und daß man die organische Phase in die Reaktionsmischung zurückführt.

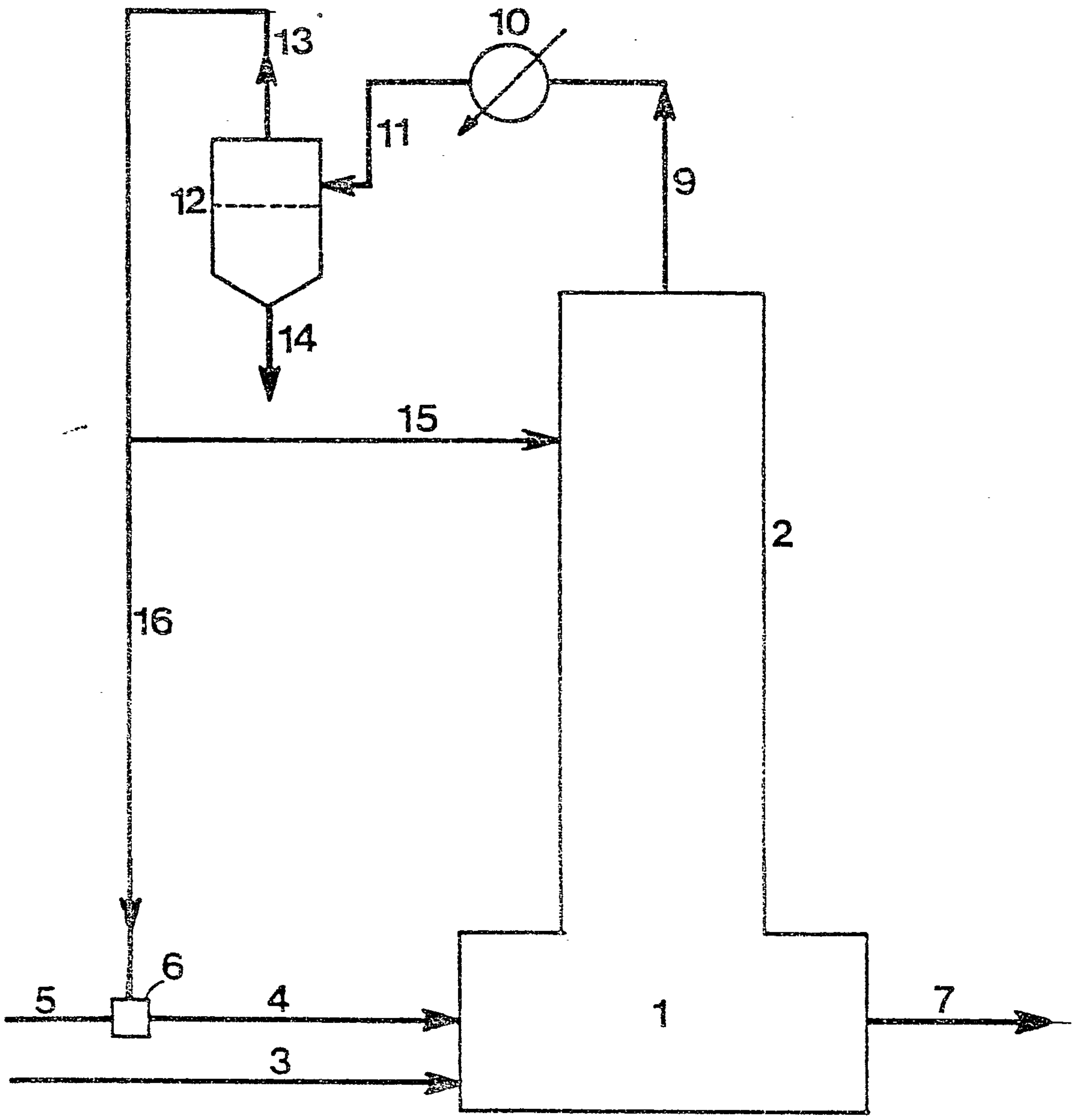
13
10
11
12
9
14
15
2
16
6
5
4
1
7
3